# EUROPEAN PATENT APPLICATION

(11) **EP 4 474 395 A1**
(43) Date of publication of application: **11.12.2024**
(21) Application number: 23177559.4
(22) Date of filing: 06.06.2023
(51) Int. Cl.: C07K 16/28, A61P 17/02, A61P 27/02, A61K 39/395

(54) **ANTI-FAS LIGAND (FASL) ANTIBODIES IN THE TREATMENT OF SJS/TEN DISEASES**

(71) Applicant: PinCell S.r.l., 20123 Milan (IT)
(72) Inventor: LOTTI, Roberta, Milan (IT); PINCELLI, Carlo, Milan (IT); AMATO, Antonino, Milan (IT); BENNETT, Brydon, Milan (IT)
(74) Representative: Weickmann & Weickmann PartmbB

(57) **Abstract**

The present invention refers to the use of antagonists of human Fas ligand (FasL; also named CD95L or Apo1L), more particularly to the use of antibodies against FasL for the prevention and/or treatment of SJS and/or TEN skin diseases.

## Description

The present invention refers to the use of antagonists of human Fas ligand (FasL; also named CD95L or CD178 or Apo1L), more particularly to the use of antibodies against FasL for the prevention and/or treatment of SJS and/or TEN skin diseases (in the following SJS/TEN).

### Background of the invention

Stevens-Johnson-Syndrome (SJS) and Toxic Epidermal Necrolysis (TEN) are severe cutaneous conditions caused by adverse reaction to specific drugs or medical substances. Both skin conditions are characterized by wide-spread erythema, epidermal necrosis, blistering and detachment of skin sections. Almost all patients with SJS and TEN have also mucosal involvement in eyes, mouth and genitals. These syndromes are considered as the same disease with different spectrums of severity. SJS represents the less severe disease spectrum and is defined by the detachment of less than 10% of the body surface area. TEN representing the more severe diseases spectrum involving skin detachment greater than 30% of the body surface area. Overlapping SJS/TEN is defined as 10-30% skin detachment.

The extensive skin detachment in SJS/TEN causes significant morbidity and mortality. The reported mortality rate in patients with SJS/TEN is estimated between 4,8% and 14,8%, respectively. Thus, SJS/TEN are considered dermatological emergencies and early recognition and successful therapy is considered lifesaving.

Although SJS/TEN is a distressing disease, there is no standard treatment for these cutaneous conditions and no approved drugs. Patients are usually treated in emergency with several drugs, such as cyclosporine, steroids, anti-TNF, IVIg or plasmapheresis (Chang HH et al., Biomedicines, 2022). None of these drugs, however, has been shown to have any beneficial impact in controlled study. Most patients receive only supportive care.

The clinical manifestation of skin exfoliation occurring in SJS/TEN is mainly due to an extensive induction of keratinocytes apoptosis and necroptosis. However, the mechanism responsible for the enhancement in SJS/TEN remains unclear.

Fas (or FasR) is a member of the TNF-receptor superfamily which, upon binding with Fas ligand (FasL), triggers apoptosis in many cell systems (Sharma *et al,* 2000). Fas-FasL interaction is involved in the pathomechanisms of several immune-inflammatory and infectious conditions. The implication of Fas-FasL pathway, for example, has been shown to be a critical mediator of keratinocytes apoptosis and acantholysis in pemphigus (Lotti et al., 2018).

Recent studies have further demonstrated that that the Fas/Fas Ligand (FasL) system plays also a crucial role in the development of SJS/TEN based on the following evidence:
(i) FasL levels in sera of patients with SJS/TEN are increased, particularly before the development of skin detachment and/or mucosal lesions (Viard et al, 1998; Abe et al., 2003, Chang et al., 2004, Murata et al, 2008);
(ii) Peripheral Blood Mononuclear Cells (PBMC) stimulated with sera of patients with SJS/TEN and/or with drug causing the SJS/TEN diseases release FasL (Abe et al. 2003);
(iii) sera from SJS/TEN patients induce keratinocyte cell death in culture (Abe et al. 2008); and
(iv) FasL is released from keratinocytes in SJS/TEN and causes cell death in neighboring keratinocytes (Abe et al. 2015).

It is an object of the present invention to provide a therapeutic agent for preventing and treating SJS/TEN. In view of the above evidence on the crucial role of FasL in SJS/TEN, the development of a new drug which blocks FasL would allow for prevention of keratinocyte apoptosis and subsequent cell detachment and acantholysis, thereby blocking the formation of severe skin lesions in SJS/TEN.

### Summary of the invention

The present disclosure provides a monoclonal antibody or an antigen-binding fragment thereof specific for human Fas ligand protein (FasL) as active agent for treating patients with Steven-Johnson-Syndrome (SJS) and/or toxic epidermal necrolysis (TEN) (SJS/TEN). The antibody is characterised by the amino acid sequences of the variable regions (CDRs) of the heavy chain variable (VH) and at least one light chain variable (VL) regions, respectively, as defined in SEQ ID Nos 1-9 disclosed herein. The use of the antibody is effective for the treatment of SJS/TEN due to its high binding affinity to human FasL.

A particular embodiment of the present invention is directed to a monoclonal antibody or an antigen-binding fragment thereof comprising a VH region having complementary determining regions CDR H1, CDR H2 and CDR H3 as assigned in SEQ ID Nos 1, 3 and 5 and a VL region having complementary determining regions CDR L1, CDR L2 and CDR L3 as assigned in SEQ ID Nos 7-9. In a more preferred embodiment of the invention the antibody has an IgG heavy chain constant region, preferably an IgG1 or IgG4 heavy chain constant region.

A further aspect of the invention relates to a nucleic acid molecule encoding a monoclonal antibody as disclosed herein or an antigen-fragment thereof for the use in the treatment of SJS/TEN.

Still a further aspect of the invention is a pharmaceutical composition comprising the antibody or antigen-binding fragment or the nucleic acid molecule disclosed herein together with one or more pharmaceutical acceptable carriers for the use in the treatment of SJS/TEN.

### Embodiments of the invention

In the following, specific embodiments of the invention are disclosed as follows:
1. A monoclonal antibody or an antigen-binding fragment thereof specific for human Fas ligand protein (FasL) comprising at least one heavy chain variable (VH) region and at least one light chain variable (VL) region, wherein said antibody or an antigen-binding fragment is selected from:
   (i) an antibody or an antigen-binding fragment comprising a VH region having complementary determining regions (CDRs) of the heavy chain CDR H1, CDR H2 and CDR H3 as follows:
      (a₁) CDR H1: Arg His Gly Ile Thr (SEQ ID NO: 1) or
      (a₂) CDR H1: Ser His Gly Ile Ser (SEQ ID NO: 2),
      (b₁) CDR H2: Trp Ile Asn Ala Tyr Asn Gly Asn Thr Asn Tyr Ala Gin Lys Val Gln Gly (SEQ ID NO: 3) or
      (b₂) CDR H2: Trp Ile Asn Ala Tyr Ser Gly Asn Thr Asn Tyr Ala Gln Lys Leu Gln Gly (SEQ ID NO: 4),
      (c₁) CDR H3: Glu Thr Met Val Arg Gly Val Pro Leu Asp Tyr (SEQ ID NO: 5) or
      (c₂) CDR H3: Glu Thr Met Val Arg Gly Val Pro Cys Asp Tyr (SEQ ID NO: 6),
      and complementary determining regions (CDRs) of the light chain CDR L1, CDR L2 and CDR L3 as follows:
         (a₃) CDR L1: Arg Ala Ser Gln Ser Val Ser Ser Ser Tyr Leu Ala (SEQ ID NO: 7),
         (b₃) CDR L2: Gly Ala Ser Ser Arg Ala Thr (SEQ ID NO: 8),
         (c₃) CDR L3: Gln Gln Tyr Gly Ser Ser Pro Trp Thr (SEQ ID NO: 9); or
   (ii) an antibody or an antigen-binding fragment competing with the antibody or antigen-binding fragment of (i) in the binding to human Fas ligand protein (FasL); for use in a method for the prevention and/or treatment of toxic epidermal necrosis (TEN) and/or Steven-Johnson-Syndrome (SJS).
2. The monoclonal antibody or an antigen-binding fragment thereof of embodiment 1 for the use of embodiment 1 comprising at least one heavy chain variable (VH) region and at least one light chain variable (VL) region, wherein said antibody or an antigen-binding fragment is selected from: an antibody or an antigen-binding fragment comprising a VH region having complementary determining regions (CDRs) of the heavy chain CDR H1, CDR H2 and CDR H3 as follows:
   (a₁) CDR H1: Arg His Gly Ile Thr (SEQ ID NO: 1),
   (b₁) CDR H2: Trp Ile Asn Ala Tyr Asn Gly Asn Thr Asn Tyr Ala Gln Lys Val Gln Gly (SEQ ID NO: 3),
   (c₁) CDR H3: Glu Thr Met Val Arg Gly Val Pro Leu Asp Tyr (SEQ ID NO: 5);
   and complementary determining regions (CDRs) of the light chain CDR L1, CDR L2 and CDR L3 as follows:
      (a₃) CDR L1: Arg Ala Ser Gln Ser Val Ser Ser Ser Tyr Leu Ala (SEQ ID NO: 7),
      (b₃) CDR L2: Gly Ala Ser Ser Arg Ala Thr (SEQ ID NO: 8),
      (c₃) CDR L3: Gln Gln Tyr Gly Ser Ser Pro Trp Thr (SEQ ID NO: 9).
3. The antibody or antigen-binding fragment thereof of embodiment 1 or 2 for the use of embodiment 1, wherein the VL region of the antibody comprises the amino acid sequence 
EIVLTQSPGTLSLSPGERATLSCRASQSVSSSYLAWYQQKPGQAPRLLIYGASSR
ATGIPDRFSGSGSGTDFTLTISRLEPEDFAVYYCQQYGSSPWTFGQGTKVEIKRT
VAAPSVFIFP(SEQ ID NO: 10)
 and the VH region of the antibody comprises the amino acid sequence 
QVQLVQSGAEVKKPGASVKVSCKASGYIFIRHGITWVRQAPGQGLEWMGWINA
YNGNTNYAQKVQGRVTMTTDKSTSTAYMELRSLRSDDAAVYYCARETMVRGVP
LDYWGQGTLVTVSSASTKGPSVFPLA (SEQ ID NO: 11),
 or 
QVQLVQSGAEVKKPGASVKVSCKASGYIFISHGISWVRQAPGQGLEWMGWINA
YSGNTNYAQKLQGRVTMTTDRSTSTAYMELRSLRSDDTAVYYCARETMVRGVP
CDYWGQGTLVTVSSASTKGPSVFPLA (SEQ ID NO: 12).
4. The antibody or antigen-binding fragment thereof of any one of embodiments 1-3 for the use of embodiment 1 recognizing the same epitope on human FasL as the antibody of claim 1 (i) or 2 (i).
5. The antibody or antigen-binding fragment thereof of any one of embodiments 1-4 for the use of embodiment 1, wherein the antibody is selected from a partially or fully human antibody, a chimeric antibody and/or a humanized antibody and wherein the antigen-binding fragment thereof is selected from a Fab, Fab' and/or F(ab')2 and/or a single chain Fv fragment.
6. The antibody or antigen-binding fragment thereof of any one of embodiments 1-5 for the use of embodiment 1, wherein the antibody has an IgG heavy chain constant region, preferably an IgG1 or IgG4 heavy chain constant region.
7. A nucleic acid molecule encoding a monoclonal antibody or an antigen-fragment thereof of any one of embodiments 1-6 for the use of embodiment 1.
8. A nucleic acid molecule of embodiment 7 for the use of embodiment 1, which is a DNA vector or an RNA molecule.
9. The antibody or antigen-binding fragment thereof of any one of any one of embodiments 1-6 or the nucleic acid molecule of any one of embodiments 7-8 for the use of embodiment 1 in a monotherapy.
10. The antibody or antigen-binding fragment thereof of any one of embodiments 1-6 or the nucleic acid molecule of any one of embodiments 7-8 for the use of embodiment 1 in combination with at least one further active ingredient effective against toxic epidermal necrosis (TEN) and/or Steven-Johnson syndrome (SJS).
11. The antibody or antigen-binding fragment thereof of any one of embodiments 1-6 or the nucleic acid molecule of any one of embodiments 7-8 for the use of embodiment 10, wherein the further active ingredient is selected from at least one of steroids, cyclosporine, IVIg, TNF inhibitors and/or plasmapheresis.
12. The antibody or antigen-binding fragment thereof of any one of embodiments 1-6 or the nucleic acid molecule of any one of embodiments 7-8 for the use of embodiment 1 in human therapy.
13. A pharmaceutical composition comprising the antibody or antigen-binding fragment of any one of embodiments 1-6 or the nucleic acid molecule of any one of embodiments 7-8 together with one or more pharmaceutical acceptable carriers for the use of embodiment 1.
14. The antibody or antigen-binding fragment thereof of any one of embodiments 1-6 or the nucleic acid molecule of any one of embodiments 7-8 or the pharmaceutical composition of embodiment 13, which is administered systemically and/or locally.

### Detailed description

The present invention refers to the treatment of SJS/TEN diseases, associated with keratinocyte apoptosis and necroptosis, by administering a FasL antagonist.

In general, FasL antagonists may be selected from anti FasL antibodies, particularly humanized or human anti FasL antibodies, nucleic acid effector molecules of Fas expression such as antisense molecules or molecules capable of RNA interference such as siRNA molecules, soluble Fas receptor molecules, antagonistic FasL muteins, and low molecular weight chemical compounds inhibiting the Fas-FasL interaction. FasL antagonists prevent keratinocyte apoptosis and subsequent cell-cell detachment (acantholysis).

The present invention thus relates to the use of at least one anti-FasL compound able to inhibit the biological effects of FasL. The expression "*inhibiting the biological effects of FasL*" used herein relates to compounds which can fully or at least substantially inhibit or neutralize the biological effects of FasL. For example, the inhibitory or neutralizing effect may be based on suppressing the binding of FasL to its natural receptor, thereby suppressing the caused signal transmissions. This can be achieved e.g., by using antibodies binding to FasL per se or soluble receptors mimicking Fas or antagonistic FasL muteins, thus blocking the binding of FasL to its cellular receptors. Alternatively, interfering with Fas or FasL expression by siRNA will block Fas/FasL system likewise.

One aspect of the present invention relates to the use of anti-FasL antibodies or an active fragment thereof as therapeutic effective agent in the prevention and/or treatment of SJS/TEN.

In the context of the present invention, the term "prevention of SJS/TEN" means the treatment of the diseases at diagnosis, e.g. in an early stage in order to prevent the progression of the diseases to a more severe spectrum of severity. The term "treatment of SJS/TEN" means that the SJS/TEN diseases is managed to lessen and/or ameliorating the symptoms of the disease, preferably to the point of curing the disease.

The antibodies are preferably chimeric, humanized or human anti-FasL antibodies. Further, the antibodies may be monovalent or multivalent and may comprise modifications such as different glycosylation pattern, or modification of the Fc region to alteration of the antibody dependent cellular cytotoxicity (ADCC) and the complement-dependent cytotoxicity (CDC). The antigen-binding fragment or derivative of the anti-FasL antibody of the invention may be a recombinant single chain antibody or single chain variable fragment. In the very preferred embodiment of the invention, the antibodies are human FasL-antibodies. If desired, the antibodies may be conjugated to effector molecules, e.g., cytostatic, cytotoxic and/or radioactive compounds.

In a first aspect, the present invention is directed to a monoclonal antibody or an antigen-binding fragment thereof specific for human Fas ligand protein (FasL) comprising at least one heavy chain variable (VH) region and at least one light chain variable (VL) region, wherein the antibody or the antigen-binding fragment comprises a VH region having complementary determining regions (CDRs) of the heavy chain CDR H1, CDR H2 and CDR H3 as follows:
(a₁) CDR H1: Arg His Gly Ile Thr (SEQ ID NO: 1) or
(a₂) CDR H1: Ser His Gly Ile Ser (SEQ ID NO: 2),
(b₁) CDR H2: Trp Ile Asn Ala Tyr Asn Gly Asn Thr Asn Tyr Ala Gln Lys Val Gln Gly (SEQ ID NO: 3) or
(b₂) CDR H2: Trp Ile Asn Ala Tyr Ser Gly Asn Thr Asn Tyr Ala Gln Lys Leu Gln Gly (SEQ ID NO: 4), and
(c₁) CDR H3: Glu Thr Met Val Arg Gly Val Pro Leu Asp Tyr (SEQ ID NO: 5) or
(c₂) CDR H3: Glu Thr Met Val Arg Gly Val Pro Cys Asp Tyr (SEQ ID NO: 6),
and complementary determining regions (CDRs) of the light chain CDR L1, CDR L2 and CDR L3 as follows:
   (a₃) CDR L1: Arg Ala Ser Gin Ser Val Ser Ser Ser Tyr Leu Ala (SEQ ID NO: 7),
   (b₃) CDR L2: Gly Ala Ser Ser Arg Ala Thr (SEQ ID NO: 8), and
   (c₃) CDR L3: Gln Gln Tyr Gly Ser Ser Pro Trp Thr (SEQ ID NO: 9);
for use in a method for the prevention and/or treatment of toxic epidermal necrosis (TEN) and/or Steven-Johnson syndrome (SJS).

In a preferred embodiment the invention relates to a monoclonal antibody or an antigen-binding fragment thereof specific for human Fas ligand protein (FasL) comprising at least one heavy chain variable (VH) region having complementary determining regions (CDRs) of the heavy chain CDR H1, CDR H2 and CDR H3 as follows:
(a₁) CDR H1: Arg His Gly lie Thr (SEQ ID NO: 1),
(b₁) CDR H2: Trp Ile Asn Ala Tyr Asn Gly Asn Thr Asn Tyr Ala Gln Lys Val Gln Gly (SEQ ID NO: 3)
(c₁) CDR H3: Glu Thr Met Val Arg Gly Val Pro Leu Asp Tyr (SEQ ID NO: 5),
and and at least one light chain variable (VL) region having complementary determining regions (CDRs) of the light chain CDR L1, CDR L2 and CDR L3 as follows:
(a₃) CDR L1: Arg Ala Ser Gln Ser Val Ser Ser Ser Tyr Leu Ala (SEQ ID NO: 7),
(b₃) CDR L2: Gly Ala Ser Ser Arg Ala Thr (SEQ ID NO: 8), and
(c₃) CDR L3: Gln Gln Tyr Gly Ser Ser Pro Trp Thr (SEQ ID NO: 9);
for use in a method for the prevention and/or treatment of toxic epidermal necrosis (TEN) and/or Steven-Johnson syndrome (SJS).

Further, the invention relates to a monoclonal antibody or an antigen-binding fragment which competes with the monoclonal antibodies or antigen-binding fragment disclosed above for the binding to human Fas ligand protein (FasL). Preferably, the antibody competes with the monoclonal antibody having heavy chain CDR sequences H1- H3 comprising the amino acid sequences of SEQ ID Nos 1-5 and light chain CDR sequences L1-L3 comprising the amino acid sequences of SEQ ID Nos 7-9 as described above. In a very preferred embodiment, the antibody competes with the monoclonal antibody having heavy chain CDR sequences H1-H3 of SEQ ID Nos 1, 3 and 5 and light chain CDR L1-L3 of SEQ ID Nos 7-9 as described above.

In certain embodiments, a competing antibody of the present invention binds the same or an overlapping epitope on the human FasL as the monoclonal antibody having CDRs as defined in SEQ ID Nos 1-9. Competition may be determined by standard assays in the art that can quantify binding affinity, relative and absolute, of the binding proteins, particularly antibody to human FasL with respect to the given reference antibody.

A determination of quantitative binding of FasL antibody to FasL protein can be conducted by one of various surface plasmon resonance (SPR) measuring platforms. Examples include Biacore and Forte Octet. Recombinant target protein, FasL (e.g., Aero Bio FAL-H5241) is tethered to a capture chip and FasL antibody is flowed over the chip while changes in molecular interactions are recorded in real time. On-rates (ka; M⁻¹ s⁻¹), off-rates (kd; s⁻¹) can be calculated by adding and removing FasL from the flow. A measure of binding affinity (KD, µM) can be determined by dividing the off-rate by the on-rate. KD values less than 10 µM are typically required for therapeutic use. KD values of 10 nM to 1 pM are optimally used.

In particular embodiments of the present invention, at least one amino acid of the above CDR1, CDR2 or CDR3 amino acid sequence of the VH and/or VL chain is replaced by another amino acid, while preserving structural integrity and epitope-binding of the antibody. These exchanges can be conservative (i.e., by a similar amino acid) or non-conservative.

In particular, at least one amino acid of the VH and VL CDR1, CDR2 or CDR3 sequences, may be replaced by a conservative amino acid substitution, i.e., a substitution of an amino acid by another amino acid with similar biochemical properties, for example a substitution of an aliphatic amino acid, e.g., Gly, Ala, Val, Leu, or Ile, for another aliphatic amino acid; a substitution of a basic amino acid, e.g. His, Lys or Arg, against another basic amino acid or against Met; a substitution of an acidic amino acid or an amide thereof, e.g., Asp, Glu, Asn or Gln, against another acidic amino acid or an amide thereof; a substitution of an aromatic amino acid, e.g., Phe, Tyr or Trp, against another aromatic amino acid. In certain preferred embodiments of the invention, 1, 2, 3, 4, or 5 amino acids of SEQ. ID NO: 1-9 are replaced by another conservative or non-conservative amino acid.

A preferred embodiment, the present invention is directed to the use of anti-FASL human antibodies, or antigen-binding portions thereof, comprising a light chain variable region and/or a heavy chain variable region as described in US 7,262,277 (SEQ ID NO 2 and SEQ ID NO 10 or 18, respectively of US 7,262,277), the content of which is herein incorporated by reference.

Accordingly, in a still further preferred embodiment of the invention the anti-FasL antibodies comprises a light chain variable region comprising a polypeptide with the amino acid sequence as follows: 
EIVLTQSPGTLSLSPGERATLSCRASQSVSSSYLAWYQQKPGQAPRLLIYGASSR
ATGIPDRFSGSGSGTDFTLTISRLEPEDFAVYYCQQYGSSPWTFGQGTKVEIKRT
VAAPSVFIFP (SEQ ID NO: 10),
 and a heavy chain variable region comprising a polypeptide with the amino acid sequence as follows: 
QVQLVQSGAEVKKPGASVKVSCKASGYIFIRHGITWVRQAPGQGLEWMGWINA
YNGNTNYAQKVQGRVTMTTDKSTSTAYMELRSLRSDDAAVYYCARETMVRGVP
LDYWGQGTLVTVSSASTKGPSVFPLA (SEQ ID NO: 11)
 or 
QVQLVQSGAEVKKPGASVKVSCKASGYIFISHGISWVRQAPGQGLEWMGWINA
YSGNTNYAQKLQGRVTMTTDRSTSTAYMELRSLRSDDTAVYYCARETMVRGVP
CDYWGQGTLVTVSSASTKGPSVFPLA (SEQ ID NO: 12).
 for use the prevention and/or treatment of toxic epidermal necrosis (TEN) and/or Steven-Johnson syndrome (SJS).

The antibody according to the present invention for the use in the prevention and/or treatment of SJS/TEN may be selected from a partially or fully human antibody, a chimeric antibody and/or a humanized antibody. Preferably, the antibody is a human antibody. Further, the antibody according to the present invention may be monospecific and/or bispecific. Alternatively, the antibody may be multivalent and multispecific. Further, the antigen-binding fragment of the antibody according to the present invention may be selected from a Fab, Fab' and/or F(ab')2 and/or a single chain Fv fragment.

In a very preferred embodiment of the invention, the antibody has an IgG heavy chain constant region, preferably an IgG1 or IgG4 heavy chain constant region. In a further preferred embodiment of the invention, the antibody has a kappa (κ) light chain.

In a still further embodiment, the invention relates to the use of the anti-hFas Ligand human antibody, or an antigen binding fragment thereof, produced by the hybridoma cell with accession number ATCC PTA-4017 and/or to the use of the anti-hFas Ligand human antibody, or an antigen binding fragment thereof, produced by the hybridoma cell with accession number ATCC PTA-4018 as described in US 7,262,277 as antibodies 3E1 and 4G11, respectively for the prevention and/or treatment of SJS/TEN. In the most preferred embodiment of the invention is directed to the use of the anti-hFas Ligand human antibody 3E1 (produced by the hybridoma cell with accession number ATCC PTA-4017), or an antigen binding fragment thereof for the prevention and/or treatment of SJS/TEN.

The hybridoma cell under Accession No. ATCC PTA-4017 and ATCC PTA-4018 have been deposited on 29 January 2002 with the American Type Culture Collection, 10801 University Boulevard, Manassas, Virginia 20110-2209 (USA).

In a finally further preferred aspect, the present invention refers to the use of a monoclonal antibody or an antigen-binding fragment thereof recognizing the same epitope of human FasL as the antibodies described above.

A still further preferred embodiment of the invention refers to a nucleic acid molecule encoding a monoclonal antibody or an antigen-fragment thereof as disclosed above for the use in the prevention and/or treatment of SJS/TEN. The nucleic acid molecule may be a DNA vector or a RNA molecule typically formulated as a lipid nanoparticle encapsulation. The nucleic acid molecule encoding the antibody of the invention when injected into a patient, is incorporated into the cells, which express the antibody into circulation. The use of DNA and RNA delivery technology in antibody therapy and, in particular, the use of mRNA encoded therapeutic antibodies, is a well-known therapeutic approach in the art (e.g., Van Hoecke and Roose, J Trans Med (2019) 17:54 and Deal at al., Vaccines (2021), 9, 2018).

In therapeutic applications, the anti-FasL antibodies of the invention, or antibody fragments thereof, are administered in an effective amount to a subject in need thereof, particularly to a human subject. The dose will depend on the specific type of antibody, the severity and stage of disease, and the route of administration.

In a preferred embodiment of the invention, the subjects affected by SJS/TEN in need of the anti-FasL antibodies of the invention are subjects which have a FasL concentration value in the serum elevated compared to an healthy subject.

Typically, the anti-FasL antibodies of the invention, or antibody fragments thereof, are administered as a pharmaceutical composition comprising the active agent and a pharmaceutically acceptable carrier or excipient. Examples of suitable carriers and excipients for formulating antibodies or antibody fragments are well-known in the art. An effective dose of a medicament of the present invention may be in the range of 0.1 µg to 100 mg, up to a total dose of about 1 g depending upon the route of administration.

Thus, a further aspect of the present invention is a pharmaceutical composition comprising an anti-FasL antibody or antigen-binding fragment thereof as described above together with one or more pharmaceutical acceptable carriers for use in a method for the prevention and/or treatment of toxic epidermal necrosis (TEN) and/or Steven-Johnson syndrome (SJS).

Depending on the stage and the severity of the disorder, the pharmaceutical composition may be administered once or several times during the course of the disorder. For example, it may be administered once or several times daily, each second day, two times weekly or weekly for a suitable period of time. The pharmaceutical composition may be administered in a single treatment cycle consisting of one or several administrations or in several treatment cycles each consisting of one or several administrations. Each treatment cycle may have a duration of one day up to several weeks, months, or even years.

In certain embodiments, the pharmaceutical composition is administered parenterally, e.g., by subcutaneous, intramuscular or intravenous injection or by infusion. In further embodiments, the pharmaceutical composition is administered locally, e.g., topically, orally, nasally or intrapulmonary, for example by inhalation as an aerosol. Preferably, the composition is administered systemically.

The anti-FasL antibodies or antibody fragments thereof may be used in a monotherapy or in a combination therapy. Thus, the anti-FasL antibodies or antibody fragments thereof may be administered alone or together with at least one further active agent effective in the treatment of SJS and/or TEN. In particular, the anti-FASL antibodies of the present invention may be used in combination with other drugs particularly selected from steroids, cyclosporine, IVIg, TNF-inhibitors or plasmapheresis.

Further, the present invention is explained in more detail by the following Figures and Examples.

### Figure Legends

Figure 1: FasL concentration of SJS/TEN serum measured by ELISA. S01 to S08 are all serum of SJS/TEN patients. The dotted line is the normal value; 124 pg/ml or less.
Figure 2: HaCaT cell viability after treatment with serum. H0A is healthy serum and S0A-S0B are SJS/TEN serum. Serum concentrations were set at 1, 5, and 10%.
Figure 3 and Figure 3a: Cell death inhibitory effect by the PC111 antibody, including statistical analysis. The PC111 antibody showed a cell death inhibitory effect in a concentration-dependent manner. NS = no serum; PC111 antibody dose is expressed in µg/mL; the "PC111 0" dose is SJS/TEN serum only. PC111 10 µg/mL, p value = 0.0159.
Figure 4 and Figure 4a: Cell death inhibitory effect by zVAD, including statistical analysis (Z-VAD-FMK, pan-caspase inhibitor), µM.
Figure 5 and Figure 5a: No induction of HaCaT cell death with serum of healthy patients, including statistical analysis.
Figure 6: Summary of the experimental design and schedules of the in vivo studies. An acetaminophen-induced SJS/TEN mouse model was constructed using PBMCs from one patient and the causative drug, acetaminophen. Effects of the PC111 antibody were compared by dividing groups into whether or not PC111 was injected.
Figure 7: Ocular manifestation at D14. The degree of hyperemia and edema of the ocular conjunctiva was evaluated and scored in a 0-3 scale. N/A = not available.
Figure 8: Histopathological findings for each of the tested mice groups. Scale bar, 200 µm (a, c), 50 µm (b, d).
Figure 9: TUNEL staining for counting dead epithelial cells. The TUNEL assay detected numerous dead epithelial cells in control group (a, b) compared with treatment group (c, d). Scale bar, 200 µm (a, c), 50 µm (b, d).
Figure 10: Ratio of positive cells for TUNEL staining to the total conjunctival cell count. The ratio of dead cells was significantly decreased in the treatment group. Significant differences were evaluated by Student's t-test (*p<0.05).

### Examples

In order to evaluate the therapeutic effect of FasL antibody on SJS/TEN *in vitro* and *in vivo* experiments have been performed.

An anti-FasL human IgG4, kappa, monospecific bivalent antibody comprising a heavy chain variable (VH) region with complementary determining regions CDR H1, CDR H2 and CDR H3 according to SEQ ID Nos 1, 3 and 5 and a light chain variable (VL) region with complementary determining regions CDR L1, CDR L2 and CDR L3 according to SEQ IDs 7-9 was used. This antibody is internally and hereafter named "*antibody PC111*"*.*

### 1. In vitro Studies

### 1.1.Materials and Methods

### 1.1.1 Cell culture

HaCaT cells, a spontaneously immortalised adult keratinocyte cell line, were purchased from COSMO BIO (Tokyo, Japan) and cultured in CnT-PR (CELLnTEC, Stauffacherstrasse, Switzerland), in an incubator at 37 °C under 5% CO2.

### 1.1.2 Generation of SJS/TEN model cell

To generate SJS/TEN model cell, HaCaT cells cultured in CnT-PR were treated with 1%, 5% and 10 % SJS/TEN serum for 24 hours. The PC111 antibody was added at the same time as serum. SJS/TEN patient sera were obtained from Niigata University Hospital.

### 1.1.3 Cell toxicity assay

Cell viability or toxicity were evaluated using Live/Dead Cell Staining Kit II (PromoCell, Sickingenstr, Germany). HaCaT cells were treated in 96-well plates then incubated at 37 °C in a 5% CO₂ incubator. Images were acquired using a Keyence BZ-X710 all-in-one fluorescence microscope (Keyence, Osaka, Japan). The number of live cell and dead cell were counted using ImageJ. Cell toxicity was calculated as following: number of dead cell/(number of dead cell and live cell).

ELISA was used to measure FasL levels present in patients' serum. Thus, we need to add this method here.

FasL levels in SJS/TEN serum were measured with an enzyme-linked immunosorbent assay (ELISA) kit (R&D systems, Minneapolis, MN).

### 1.2 Results

### 1.2.1 FasL levels were high in SJS/TEN patient serum

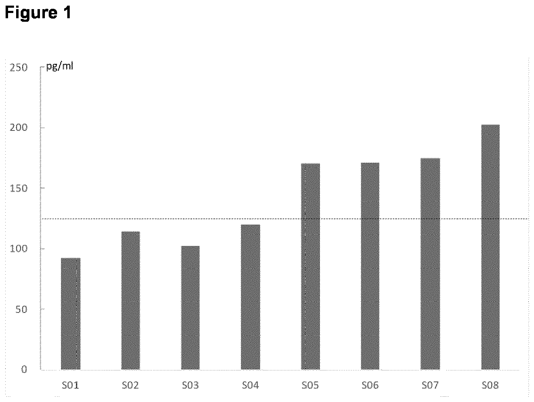

Before performing the *in vitro* assays using serum samples from the patients with SJS/TEN, we measured FasL level in SJS/TEN serum by an enzyme-linked immunosorbent assay (ELISA). FasL level was elevated in 4 out of 8 SJS/TEN serum (normal value: 124 pg/ml or less) (Figure 1). This experiment was performed only once.

### 1.2.2 Anti-FasL antibody suppress cell death from SJS/TEN serum

SJS/TEN patient serum and serum from a normal subject were tested at 1%, 5% and 10% concentration for 24 hours to observe loss of cell viability.

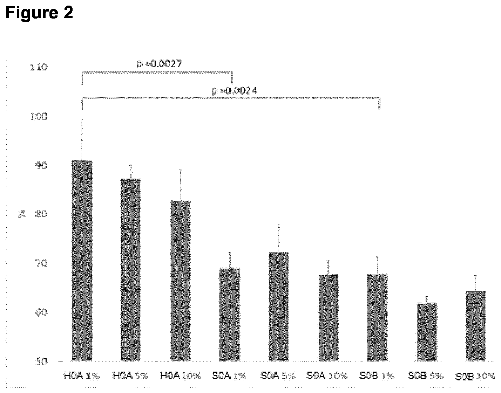

We confirmed that SJS/TEN serum can induce cell death in HaCaT cells. Compared with healthy serum, SJS/TEN serum significantly induced cell death, even at low concentration of SJS/TEN serum (1%) (S0A 1%; p=0.0027, S0B 1%; p=0.0024, respectively.) (Figure 2). Viability decreased from 80-90% with normal serum to 50-60% with patient serum. It was found that even a serum concentration of 1% can sufficiently induce cell death.

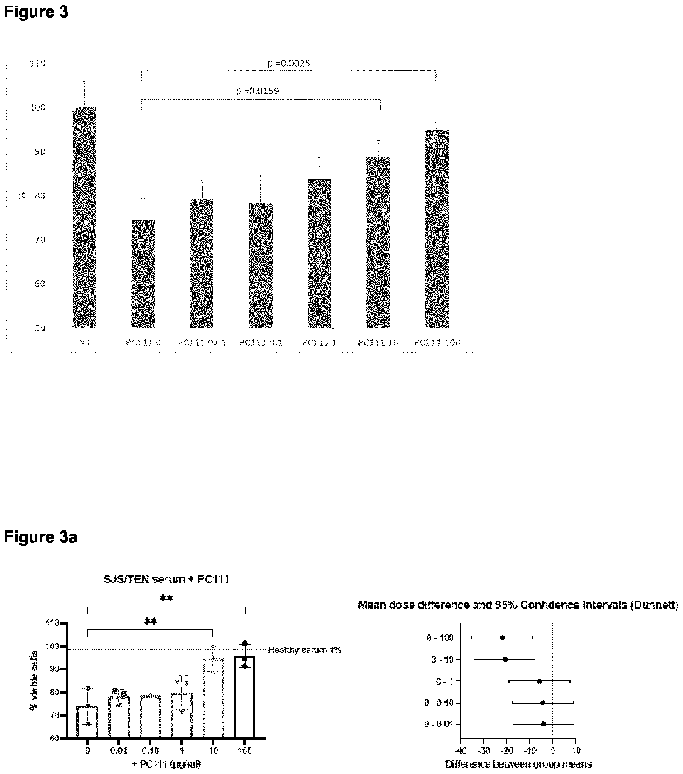

We then confirmed the efficacy of the PC111 antibody using serum S08 (as analysed in Figure 1), which had the highest FasL concentration, i.e. in the range from 90 to 200 pg/mL (cf. Figure 1). The serum with the highest level of FasL was then used for a PC111 titration experiment. As a result, it was found that SJS/TEN serum S08 induced HaCaT cell death, and that the PC111 antibody suppressed the SJS/TEN serum S08-induced cell death in a dose-dependent manner (Figures 3 and 3a). Cell death was significantly suppressed when the concentration of the PC111antibody was 10-100 µg/ml (PC111 10 µg/ml; p=0.0025, 100 µg/ml; p=0.0159, respectively). This experiment was repeated 3 times with similar results (see statistical analysis in Figure 3a).

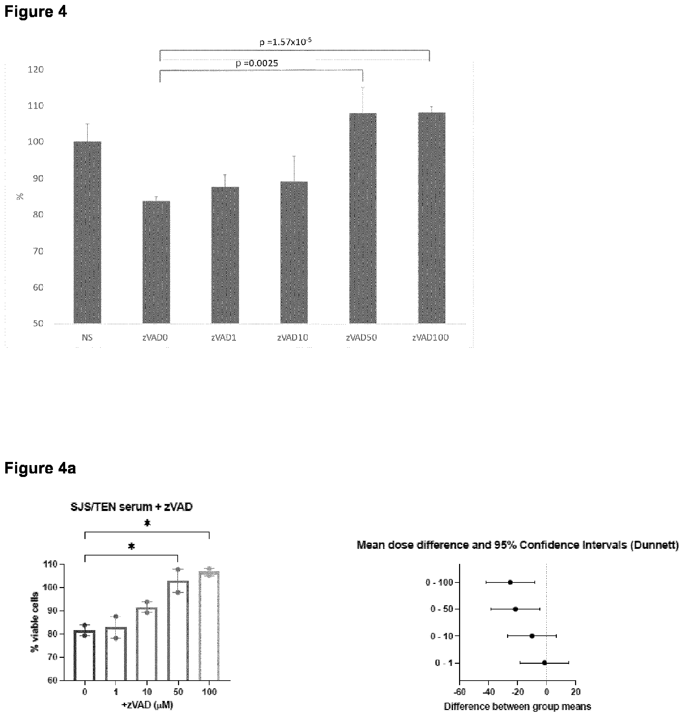

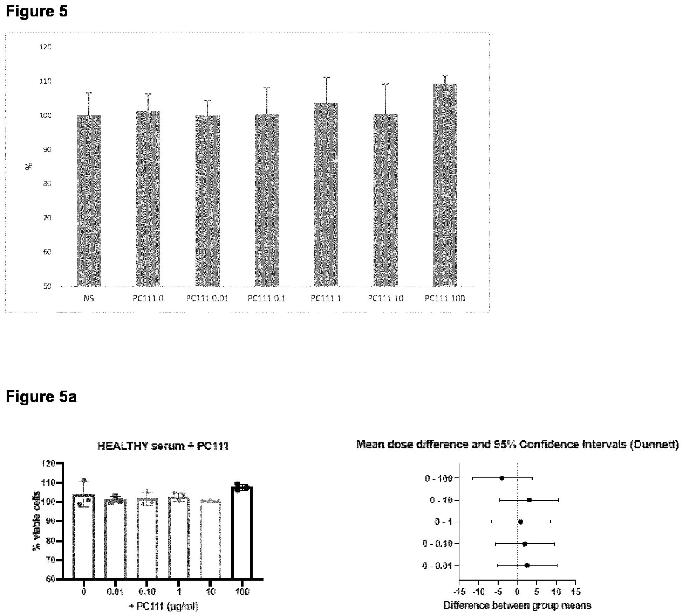

In addition, we conducted an experiment using zVAD, a pan caspase inhibitor, as a pathway comparison control for the PC111 antibody, and confirmed that it has a significant effect of suppressing apoptosis (zVAD 50µM; p=0.0025, 100 µM; p=1.57×10⁻⁵) (Figures 4 and 4a). Healthy serum did not induce HaCaT cell death (Figures 5 and 5a). This experiment was repeated 3 times with same results.

Since cell death is suppressed by zVAD, it is thought that apoptosis, which is caspase-dependent cell death, has been mainly observed in this experimental system using serum of patients with SJS/TEN. Furthermore, the experimental results suggest that the PC111 antibody has a therapeutic effect on SJS/TEN through suppressing apoptosis of keratinocytes due to Fas-FasL interaction.

In sum, the *in vitro* studies showed that SJS/TEN serum can cause cell death in HaCaT cells *in vitro* and that the PC111 antibody can dose dependently suppress apoptosis of HaCaT cells.

### 2. In vivo Studies

A validated mouse model of SJS/TEN that uses transfer of PBMCs from SJS/TEN patients (Saito et al. 2013) was used for evaluating the in vivo efficacy of the anti-FasL antibody PC111.

As reported in the following, the in vivo study results confirmed that the PC111 antibody according to the invention is an effective therapeutic agent for use in the treatment of SJS/TEN.

### 2.1 Methods

### 2.1.1 Mice

Immunocompromised NOD/Shi-scid, IL-2Rγnull (NOG) mice at 6 weeks of age were purchased from In-Vivo Science Inc. (Tokyo, Japan). Mice were included in the experiments with n=3 in each of the PC111 treatment and control groups. All the animal experiments were performed under the approval of the ethics committee for animal studies of Niigata University.

### 2.1.2 SJS/TEN mouse model using patients' PBMCs

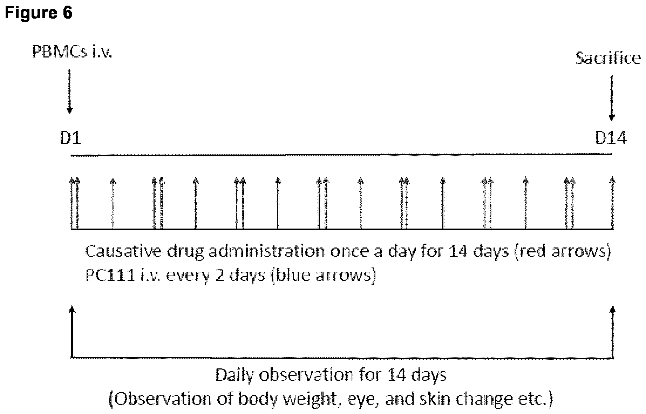

The experimental design and schedules are summarised in Fig. 6.

Peripheral Blood Mononuclear Cells (PBMCs) were obtained from a patient who had recovered from SJS/TEN. The patients received no systemic glucocorticoids at the time of PBMC collection. PBMCs (2×10⁶) were injected intravenously into the NOG mice (n=3 in each group), followed by oral administration of the causative drugs (acetaminophen, 1.5 mg/100 µl).

The dosage used in the model was based on milligrams per kilogram of body weight, converted from the adult human normal dose. The drug was administered to the mice once daily. In addition, it was confirmed that the dosage was under the median lethal dose in mice. Drug dosage was estimated by dose conversion by body weight. Thus, PC111 antibody (100 µg/100ul) or PBS (100ul) as control was administered intravenously every second day from day 1 (D1, 100 µl). Mice were observed for 14 days. Any changes of the skin, eyes, and mucosa, such as skin colour or mucous haemorrhage was checked daily. Endpoints were collected at day 14 (D14). General anesthesia was carried out on day 14, and the ocular conjunctiva was dislocated and evaluated in detail for findings of hyperemia and edema. The mice were then sacrificed, and the eyeballs were collected as specimens. Ocular lesions were investigated by means of histopathologic examination and immunohistochemical staining.

### 2.1.3 Immunohistochemistry

Terminal deoxynucleotidyl transferase-mediated dUTP nick end labeling (TUNEL) is a method for detecting apoptotic cells with DNA fragmentation by labeling the terminal end of nucleic acids.

The TUNEL assay was performed according to the manufacturer's protocol (Takara Bio, Shiga, Japan). The ocular conjunctiva was observed macroscopically and the number of dead cells was macroscopically counted for all 3 animals per group. Significant differences in the ratio of dead cells between the PC111 and control groups were evaluated by Student's t-test. p < 0.05 was defined as a significant difference.

### 2.2 Results

Daily observations of skin manifestation were made throughout the experiment. No remarkable changes of skin were observed until D14. One mouse in the control treatment group died at day 12 (D12) of unknown cause.

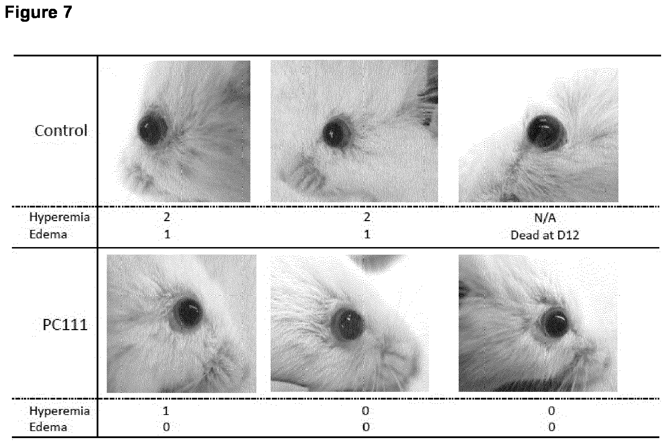

The ocular conjunctiva was observed in detail by dislocating eyeballs before mice sacrifice on D14 (Figure7). In the control group (n=2), hyperemia of the ocular conjunctiva was noticeable (score of 2, in a 0-3 scale) and mild edema (score of 1, in a 0-3 scale) was also observed. In the PC111-treated group (n=3), only one mouse showed mild conjunctival hyperemia (score of 1, in a 0-3 scale), without edema, while the other two PC111-tretaed mice showed neither hyperemia nor edema.

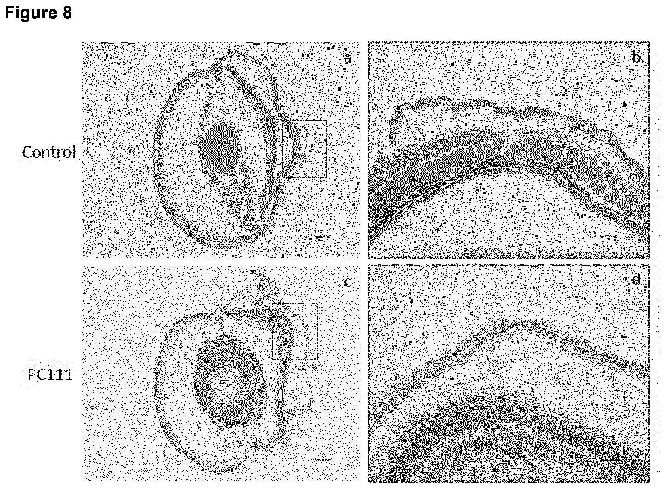

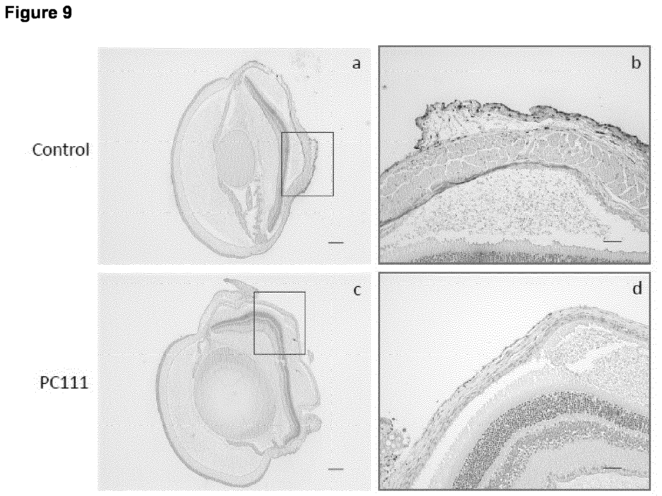

In a further step, the recovered eyeballs were evaluated histologically (Figure 8). Hematoxylin-eosin staining showed mild edema of the ocular conjunctiva in both groups. Dyskeratosis of epithelial cells, such as those seen in patients with SJS/TEN, were not obvious in either mouse group. Therefore, TUNEL staining was performed to objectively assess the number of dead cells (Figure 9).

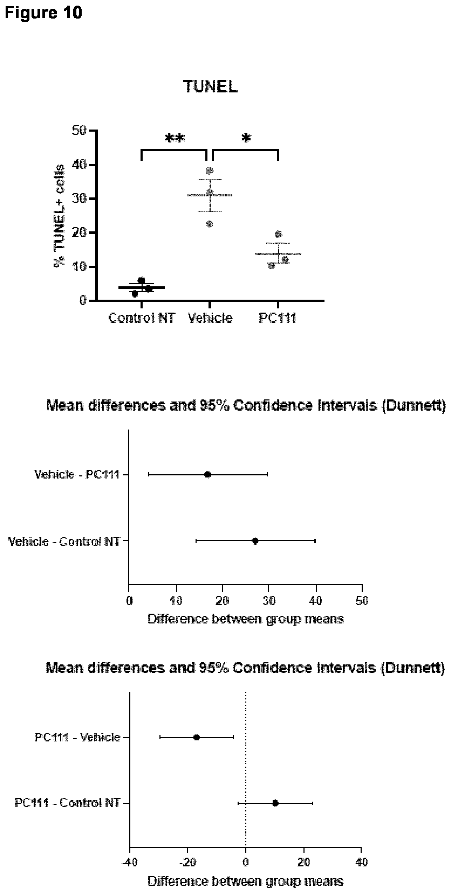

The results showed a large number of dead cells in the epithelial cells in the control group and only a few in the PC111-treated group. The ratio of the number of TUNEL-positive cells to the total conjunctival cell count was calculated for all mice, and significant differences were evaluated by One-way ANOVA followed by Dunnett's multiple comparisons test (Figure 10). The results showed that the PC111-treated group had a significantly lower ratio of TUNEL-positive cells than the vehicle-treated group (p=0.0164) and no difference with the untreated control group (p=0.1074). In the present experiment, hyperemia and edema of the ocular conjunctiva were clearly suppressed and TUNEL staining showed that TUNEL-positive cells significantly decreased in the PC111-treatemnt group.

Thus, taken together the results of the *in vivo* model study, provide evidence that the PC111 antibody is effective to decrease inflammatory symptoms and in inhibiting epithelial cell apoptosis death of the epithelium in the posterior conjunctiva of the eye in the ocular conjunctiva mouse model of SJS/TEN.

### References

1) Viard I, et al. Inhibition of toxic epidermal necrolysis by blockade of CD95 with human intravenous immunoglobulin. Science 1998; 282:490-3.
2) Abe R, et al. Toxic epidermal necrolysis and Stevens-Johnson syndrome are induced by soluble Fas ligand. Am J Pathol 2003; 162: 1515-20.
3) Chang HY, et al. Kinetics and specificity of fas ligand induction in toxic epidermal necrolysis. Arch Dermatol 2004; 140: 242-4.
4) Abe R, et al. Toxic epidermal necrolysis, and Stevens-Johnson syndrome: soluble Fas ligand involvement in the pathomechanisms of these diseases. J Dermatol Sci 2008; 52: 151-9.
5) Abe R, et al. Immunological response in Stevens-Johnson syndrome and toxic epidermal necrolysis. J Dermatol 2015; 42: 42-8.
6) Puviani M, et al. Fas ligand in pemphigus sera induces keratinocyte apoptosis through the activation of caspase-8. J Invest Dermatol 2003; 120: 164-7.
7) Lotti R, et al. Soluble Fas Ligand Is Essential for Blister Formation in Pemphigus. Front Immunol. 2018; 9: 370.
8) Chang HH. et al, A Review of the Systemic Treatment of Stevens-Johnson Syndrome and Toxic Epidermal Necrolysis, Biomedicines, 2022, 10, 2105
9) Sharma K et al., Death the Fas way: regulation and pathophysiology of Fas and its ligand. Pharmacol Ther. 88:333-347, 2000
10) Saito N, et al. Stevens-Johnson syndrome/toxic epidermal necrolysis mouse model generated by using PBMCs and the skin of patients. J Allergy Clin Immunol 2013; 131: 434-41.
11) Saito N, et al. An annexin A1-FPR1 interaction contributes to necroptosis of keratinocytes in severe cutaneous adverse drug reactions. Sci Transl Med 2014; 6: 245ra95.
12) Zhang S, et al. Biologic TNF-alpha inhibitors in the treatment of Stevens-Johnson syndrome and toxic epidermal necrolysis: a systemic review. J Dermatolog Treat. 2020; 31: 66-73.
13) Van Hoecke and Roose, "How mRNA therapeutics are entering the monoclonal antibody filed", J Trans Med (2019) 17:54
14) Deal at al., "Advancements in mRNA Encoded Antibodies for Passive Immunotherapy", Vaccines (2021), 9, 2018

## Claims

1. A monoclonal antibody or an antigen-binding fragment thereof specific for human Fas ligand protein (FasL) comprising at least one heavy chain variable (VH) region and at least one light chain variable (VL) region,
wherein said antibody or an antigen-binding fragment is selected from:
(i) an antibody or an antigen-binding fragment comprising a VH region having complementary determining regions (CDRs) of the heavy chain CDR H1, CDR H2 and CDR H3 as follows:
(a₁) CDR H1: Arg His Gly Ile Thr (SEQ ID NO: 1) or
(az) CDR H1: Ser His Gly Ile Ser (SEQ ID NO: 2),
(b₁) CDR H2: Trp Ile Asn Ala Tyr Asn Gly Asn Thr Asn Tyr Ala Gln Lys Val Gln Gly (SEQ ID NO: 3) or
(b₂) CDR H2: Trp Ile Asn Ala Tyr Ser Gly Asn Thr Asn Tyr Ala Gln Lys Leu Gln Gly (SEQ ID NO: 4),
(c₁) CDR H3: Glu Thr Met Val Arg Gly Val Pro Leu Asp Tyr (SEQ ID NO: 5) or
(c₂) CDR H3: Glu Thr Met Val Arg Gly Val Pro Cys Asp Tyr (SEQ ID NO: 6), and
complementary determining regions (CDRs) of the light chain CDR L1, CDR L2 and CDR L3 as follows:
(a₃) CDR L1: Arg Ala Ser Gln Ser Val Ser Ser Ser Tyr Leu Ala (SEQ ID NO: 7),
(b₃) CDR L2: Gly Ala Ser Ser Arg Ala Thr (SEQ ID NO: 8),
(c₃) CDR L3: Gln Gln Tyr Gly Ser Ser Pro Trp Thr (SEQ ID NO: 9);
or
(ii) an antibody or an antigen-binding fragment competing with the antibody or antigen-binding fragment of (i) in the binding to human Fas ligand protein (FasL);
for use in a method for the prevention and/or treatment of toxic epidermal necrosis (TEN) and/or Steven-Johnson-Syndrome (SJS).

2. The monoclonal antibody or an antigen-binding fragment thereof of claim 1 for the use of claim 1 comprising at least one heavy chain variable (VH) region and at least one light chain variable (VL) region,
wherein said antibody or an antigen-binding fragment is selected from:
an antibody or an antigen-binding fragment comprising a VH region having complementary determining regions (CDRs) of the heavy chain CDR H1, CDR H2 and CDR H3 as follows:
(a₁) CDR H1: Arg His Gly Ile Thr (SEQ ID NO: 1);
(b₁) CDR H2: Trp Ile Asn Ala Tyr Asn Gly Asn Thr Asn Tyr Ala Gln Lys Val Gln Gly (SEQ ID NO: 3);
(c₁) CDR H3: Glu Thr Met Val Arg Gly Val Pro Leu Asp Tyr (SEQ ID NO: 5);
and complementary determining regions (CDRs) of the light chain CDR L1, CDR L2 and CDR L3 as follows:
(a₃) CDR L1: Arg Ala Ser Gln Ser Val Ser Ser Ser Tyr Leu Ala (SEQ ID NO: 7),
(b₃) CDR L2: Gly Ala Ser Ser Arg Ala Thr (SEQ ID NO: 8),
(c₃) CDR L3: Gln Gln Tyr Gly Ser Ser Pro Trp Thr (SEQ ID NO: 9).

3. The antibody or antigen-binding fragment thereof of claim 1 or 2 for the use of claim 1, wherein the VL region of the antibody comprises the amino acid sequence 
EIVLTQSPGTLSLSPGERATLSCRASQSVSSSYLAWYQQKPGQAPRLLIY
GASSRATGIPDRFSGSGSGTDFTLTISRLEPEDFAVYYCQQYGSSPWTFG
QGTKVEIKRTVAAPSVFIFP (SEQ ID NO: 10)
 and the VH region of the antibody comprises the amino acid sequence 
QVQLVQSGAEVKKPGASVKVSCKASGYIFIRHGITWVRQAPGQGLEWMG
WINAYNGNTNYAQKVQGRVTMTTDKSTSTAYMELRSLRSDDAAVYYCAR
ETMVRGVPLDYWGQGTLVTVSSASTKGPSVFPLA (SEQ ID NO: 11)
 or

QVQLVQSGAEVKKPGASVKVSCKASGYIFISHGISWVRQAPGQGLEWMG
WINAYSGNTNYAQKLQGRVTMTTDRSTSTAYMELRSLRSDDTAVYYCAR
ETMVRGVPCDYWGQGTLVTVSSASTKGPSVFPLA (SEQ ID NO: 12).

4. The antibody or antigen-binding fragment thereof of any one of claims 1-3 for the use of claim 1 recognizing the same epitope on human FasL as the antibody of claim 1 (i) or 2 (i).

5. The antibody or antigen-binding fragment thereof of any one of claims 1-4 for the use of claim 1, wherein the antibody is selected from a partially or fully human antibody, a chimeric antibody and/or a humanized antibody and wherein the antigen-binding fragment thereof is selected from a Fab, Fab' and/or F(ab')2 and/or a single chain Fv fragment.

6. The antibody or antigen-binding fragment thereof of any one of claims 1-5 for the use of claim 1, wherein the antibody has an IgG heavy chain constant region, preferably an IgG1 or IgG4 heavy chain constant region.

7. A nucleic acid molecule encoding a monoclonal antibody or an antigen-fragment thereof of any one of claims 1-6 for the use of claim 1.

8. A nucleic acid molecule of claim 7 for the use of claim 1, which is a DNA vector or an RNA molecule.

9. The antibody or antigen-binding fragment thereof of any one of any one of claims 1-6 or the nucleic acid molecule of any one of claims 7-8 for the use of claim 1 in a monotherapy.

10. The antibody or antigen-binding fragment thereof of any one of claims 1-6 or the nucleic acid molecule of any one of claims 7-8 for the use of claim 1 in combination with at least one further active ingredient effective against toxic epidermal necrosis (TEN) and/or Steven-Johnson syndrome (SJS).

11. The antibody or antigen-binding fragment thereof of any one of claims 1-6 or the nucleic acid molecule of any one of claims 7-8 for the use of claim 10, wherein the further active ingredient is selected from at least one of steroids, cyclosporine, IVIg, TNF inhibitors and/or plasmapheresis.

12. The antibody or antigen-binding fragment thereof of any one of claims 1-6 or the nucleic acid molecule of any one of claims 7-8 for the use of claim 1 in human therapy.

13. A pharmaceutical composition comprising the antibody or antigen-binding fragment of any one of claims 1-6 or the nucleic acid molecule of any one of claims 7-8 together with one or more pharmaceutical acceptable carriers for the use of claim 1.

14. The antibody or antigen-binding fragment thereof of any one of claims 1-6 or the nucleic acid molecule of any one of claims 7-8 or the pharmaceutical composition of claim 12, which is administered systemically and/or locally.
